# EUROPEAN PATENT APPLICATION

(11) **EP 1 479 351 A2**
(43) Date of publication of application: **24.11.2004**
(21) Application number: 04012099.0
(22) Date of filing: 21.05.2004
(51) Int. Cl.: A61B 17/32, A61M 25/12

(54) **Balloon catheter for detaching mucosa during EMR**

(30) Priority: 23.05.2003 JP 2003146478
(71) Applicant: Yukinobu, Takimoto, Kobe-shi, Hyogo-ken 657-0011 (JP); Kaigen Co., Ltd., Osaka-shi, Osaka 541-0045 (JP); SEKISUI CHEMICAL CO., LTD., Osaka-shi, Osaka 530-8565 (JP)
(72) Inventor: Takimoto, Yukinobu, Kobe-shi Hyogo-ken 657-0011 (JP)
(74) Representative: HOFFMANN - EITLE

(57) **Abstract**

A treatment instrument for EMR and an EMR device are provided for excising a lesion portion while reducing complications and burdens to patients in an endoscopic mucosal resection. A separating and swelling member (110) is included, so that a portion to be dissociated (5a) including the lesion portion (4), and a non-affected portion (2b) are separated and dissociated from each other by the separating and swelling member, and the lesion portion is swollen. The treatment instrument for EMR (100) and the EMR device (150) enable the endoscopic mucosal resection to be carried out more easily as compared with the conventional art, thereby greatly shortening an operation time, improving safety of the operation and moreover, reducing the burden of patients in comparison with the conventional art.

## Description

### BACKGROUND OF THE DISCLOSURE

The present invention relates to a treatment instrument for EMR used for EMR (Endoscopic Mucosal Resection), and an EMR device equipped with the treatment instrument for EMR.

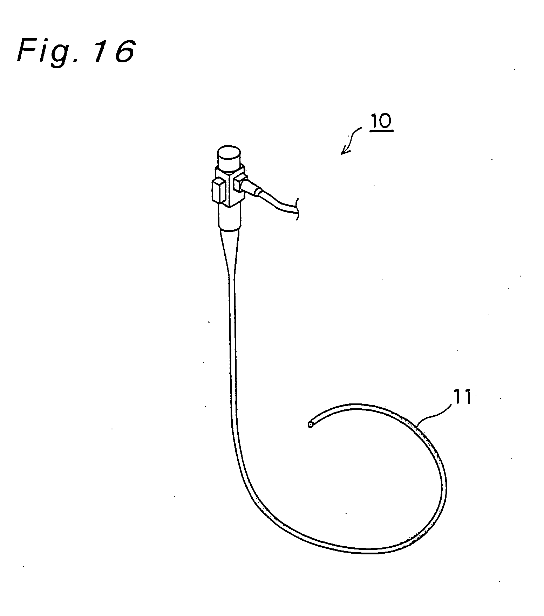

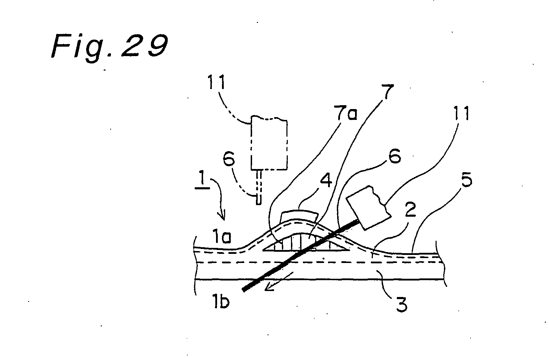

These days, to malignant and benign lesion portions within mucous membranes in a body cavity such as the esophagus, stomach, small intestine, large intestine or the like an endoscopic mucosal resection is carried out by inserting a diathermy knife or the like as a kind of an electric knife through a tube 11 into the body cavity with the use of an endoscope 10 such as shown in Fig. 16, and excising the lesion portions by the diathermy knife or the like without executing ventrutomy. According to the endoscopic mucosal resection as above, conventionally, a pharmaceutical solution such as physiological saline, 5% glycol solution, glycerol epinephrine solution, 0.5% hyaluronic acid or the like is injected to a submucosal layer 2 present under a mucosal layer 5 where a lesion portion 4 is present as shown in Fig. 29, and the mucosal layer 5 including the lesion portion 4 and the submucosal layer 2 are swollen. Then the mucosal layer 5 including the lesion portion 4, the submucosal layer 2 and a connective tissue 7a in the submucosal layer 2 present at the pharmaceutical solution injected part 7 are excised from a side face of the swollen part by the diathermy knife 6 or the like (for example, referring to a publication of unexamined Japanese application No. 2001-192336). In Fig. 29, a reference numeral 1 represents the body cavity, 1a being the body cavity inside and 1b being the body cavity outside, a reference numeral 3 is a muscular layer, and a reference numeral 6 is the diathermy knife or the like.

### SUMMARY OF THE DISCLOSURE

In the above-described conventional method, the injected pharmaceutical solution is easy to absorb in the submucosal layer 2, whereby the swelling is easily lost. Furthermore, the injected pharmaceutical solution flows out of the mucous membrane at the same time as the start of cutting the mucosal layer 5 and the submucosal layer 2, and the swelling is lost in some cases. As a result, the submucosal layer 2 and the muscular layer 3 below the submucosal layer 2 cannot be spaced fully, which may lead to the danger of perforation at the time of cutting and also the danger of leaving the lesion portion 4 without being removed. While the loss of the pharmaceutical solution makes it necessary to swell the lesion portion 4 by a plurality of the number of times during the treatment, it is difficult to swell the submucosal layer 2 by injecting the pharmaceutical solution into the same layer within the submucosal layer 2 as that of the first swelling operation for the second time and afterward, and consequently the submucosal layer is often swollen from a much deeper layer. The treatment is possibly accompanied with the complication of the perforation and bleeding, and the lesion portion 4 is left without removed, as in Fig. 29.

In addition, a lens for imaging is present at a leading end of the endoscope. For facilitating visual recognition and treatment of the lesion portion 4, it is ideal to dispose the leading end of the endoscope above the lesion portion 4 as indicated by a phantom line in'Fig. 29 and to excise the mucosal layer 5 including the lesion portion 4 and the submucosal layer 2 from above. However, according to the conventional pharmaceutical solution injection method, since the connective tissue 7a which connects an upper and lower parts of the pharmaceutical solution injected part 7 remains in the pharmaceutical solution injected part 7, the mucosal layer 5 including the lesion portion 4 and the submucosal layer 2 should be excised while the above connective tissue 7a is being cut. Under the circumstances in the conventional art as above, the mucosal layer 5 including the lesion portion 4 and the submucosal layer 2 cannot help but being excised from the side face of the swollen part, with the leading end of the endoscope being disposed at the side face of the swollen part as represented by a solid line in Fig. 29. Visual recognition of the lesion portion 4 is obstructed in many cases if the leading end of the endoscope is disposed at the side face of the swollen part, and the difficulties in visual recognition result in the complication of the perforation and bleeding, and leaving the lesion portion 4 without being removed in some cases.

The present invention is devised to solve the above problem, and has for its object to provide a treatment instrument for EMR for excising lesion portions while reducing complications and burdens to patients in an endoscopic mucosal resection, and an EMR device with the treatment instrument for EMR.

For accomplishing the above objective, the present invention is configured as described below.

Specifically, a treatment instrument for EMR in a first aspect of the present invention includes a separating and swelling member configured to be inserted in a submucosal layer located below a lesion portion present at a mucosal layer of a body cavity after inserted in the body cavity through an endoscope so as to separate and dissociate a portion to be dissociated where the lesion portion is present and a non-affected portion under the portion to be dissociated without including the lesion portion from each other, and also swell the lesion portion; and
a first introduction member having the separating and swelling member connected to a leading end of the first introduction member, which is configured to support the insertion of the separating and swelling member into the submucosal layer.

The separating and swelling member can be formed in the shape of a bag and of a material which expands by a gas or a liquid supplied through the first introduction member. Moreover, the separating and swelling member can be designed to have a size for swelling the lesion portion by the expansion.

The separating and swelling member can also be formed of a material with a heat resistance capable of resisting heat at the time of excising the swollen lesion portion.

The treatment instrument for EMR can be equipped with an installation and removal mechanism for separating the first introduction member from the separating and swelling member while the swelling state of the lesion portion by the separating and swelling member is maintained.

The treatment instrument for EMR can be configured to further include an insertion member as a member for facilitating the insertion of the separating and swelling member into the submucosal layer, which is inserted in the submucosal layer and separates the portion to be dissociated where the lesion portion is present and the non-affected portion under the portion to be dissociated without including the lesion portion from each other, thereby forming an injection part where the separating and swelling member is to be inserted; and a second introduction member with the insertion member fitted to its leading end, which supports the insertion of the insertion member into the submucosal layer.

The treatment instrument for EMR can be so constituted that the insertion member is integrally formed with the separating and swelling member, and the first introduction member and the second introduction member are integrally formed into one body.

An EMR device in a second aspect of the present invention includes the treatment instrument for EMR of the above first aspect, and an endoscope to be inserted in the body cavity from the body outside for guiding the treatment instrument for EMR passed therethrough into the body cavity.

In the second aspect, the EMR device can be further equipped with an expansion tool which is connected at the body outside to the first introduction member which is to support the separating and swelling member as a guiding member that constitutes the treatment instrument for EMR and swells the lesion portion present at the mucosal layer of the body cavity, and to inject the gas or the liquid to the separating and swelling member, whereby the separating and swelling member is expanded for swelling the lesion portion.

In the second aspect, the EMR device can also be configured to include an insertion member as a member for facilitating the insertion of the separating and swelling member into the submucosal layer, which is inserted in the submucosal layer and separates the portion to be dissociated where the lesion portion is present and the non-affected portion under the portion to be dissociated without including the lesion portion from each other, thereby forming an injection part where the separating and swelling member is to be inserted; and an expansion tool connected at the body outside to the first introduction member which is to support the separating and swelling member as a guiding member that constitutes the treatment instrument for EMR and swells the lesion portion present at the mucosal layer of the body cavity, and to inject the gas or the liquid to the separating and swelling member, whereby the separating and swelling member is expanded for swelling the lesion portion, while said expansion tool is also connected at the body outside to a second introduction member which is to support the insertion member as a guiding member that constitutes the treatment instrument for EMR and to inject a gas or a liquid to the insertion member, whereby the insertion member is expanded.

According to the treatment instrument for EMR of the first aspect and the EMR device of the second aspect of the present invention as above, the separating and swelling member is included, so that the portion to be dissociated including the lesion portion, and the non-affected portion without including the lesion portion located under the portion to be dissociated are separated and dissociated from each other, and moreover the lesion portion is swollen by the separating and swelling member. Therefore, the treatment instrument for EMR and the EMR device can prevent a situation that the swelling is disappeared simultaneously with the start of cutting the mucosal layer as in the conventional art, and the need of swelling the affected portion by a plurality of the number of times is eliminated. The possibility of generation of the complication such as the perforation and bleeding, and leaving the lesion portion without removed are nearly avoided. Thus the treatment instrument for EMR and the EMR device enable easier and safer endoscopic mucosal resection in comparison with the conventional art, can greatly shorten an operation time in comparison with the conventional art, and can reduce the burden of patients.

The separating and swelling member exerts the separating and dissociating action, whereby the need of separating and cutting a wide range by, e.g., a diathermy knife or the like so as to preliminarily form a part where the separating and swelling member is to be inserted in the submucosal layer is eliminated. The danger of perforation, bleeding, and leaving the lesion portion without removed, etc. associated with the cutting operation can be avoided.

In the case of separating and dissociating the portion to be dissociated and the non-affected portion from each other by the separating and swelling member as above, it is difficult in some cases to insert the large separating and swelling member into the submucosal layer located below the lesion portion at a time. For solving the problem, the separation and dissociation between the portion to be dissociated and the non-affected portion is carried out beforehand for a part of the submucosal layer with the use of the insertion member which facilitates the insertion of the separating and swelling member into the submucosal layer, and the injection part for the separating and swelling member is formed. The insertion and the swelling operation of the separating and swelling member are thus facilitated, so that the treatment becomes easy and moreover, the danger of perforation, bleeding, leaving the lesion portion without removed, and the like can be avoided.

### BRIEF DESCRIPTION OF THE DRAWINGS

These and other objects and features of the present invention will become clear from the following description taken in conjunction with the preferred embodiment thereof with reference to the accompanying drawings in which:
Fig. 1 is a diagram showing a treatment instrument for EMR in an embodiment of the present invention;
Fig. 2 is a diagram showing another example of the treatment instrument for EMR in the embodiment of the present invention;
Fig. 3 is a diagram showing a different example of the treatment instrument for EMR of the embodiment of the present invention, indicative of an example having an installation and removal mechanism;
Fig. 4 is a diagram showing a state with a first introduction member removed from the treatment instrument for EMR shown in Fig. 3;
Fig. 5 is a diagram showing a leading end of an endoscope installed in an EMR device of an embodiment of the present invention;
Fig. 6 is a diagram showing a lesion portion and its vicinity in a body cavity;
Fig. 7 is a diagram for explaining an endoscopic mucosal resection carried out with the use of the EMR device having the treatment instrument for EMR in the embodiment of the present invention, showing an injection state into a submucosal layer;
Fig. 8 is a diagram for explaining the endoscopic mucosal resection carried out with the use of the EMR device having the treatment instrument for EMR in the embodiment of the present invention, showing a state with the treatment instrument for EMR inserted in the submucosal layer;
Fig. 9 is a diagram for explaining the endoscopic mucosal resection carried out with the use of the EMR device having the treatment instrument for EMR in the embodiment of the present invention, showing a state in which a separating and swelling member inserted in the submucosal layer is expanded;
Fig. 10 is a diagram for explaining the endoscopic mucosal resection carried out with the use of the EMR device having the treatment instrument for EMR in the embodiment of the present invention, explanatory of excision of the lesion portion;
Fig. 11 is a diagram for explaining the endoscopic mucosal resection carried out with the use of the EMR device having the treatment instrument for EMR in the embodiment of the present invention, explanatory of excision of the lesion portion;
Fig. 12 is a diagram for explaining the endoscopic mucosal resection carried out with the use of the EMR device having the treatment instrument for EMR in the embodiment of the present invention, showing the lesion portion and an excision section in a plane;
Fig. 13 is a diagram showing a modified example of the first introduction member shown in Fig. 1, indicative of an example having a guide member attached to the first introduction member;
Fig. 14 is a diagram showing a modified example of the first introduction member shown in Fig. 1;
Fig. 15 is a diagram for explaining a modified example of the endoscopic mucosal resection shown in Figs. 10 and 11, explanatory of excision of the lesion portion;
Fig. 16 is a perspective view showing the whole of an endoscope;
Fig. 17A is a diagram showing a shape example when the separating and swelling member shown in Figs. 1-3 is expanded;
Fig. 17B is a diagram showing a shape example when the separating and swelling member shown in Figs. 1-3 is expanded;
Fig. 17C is a diagram showing a shape example when the separating and swelling member shown in Figs. 1-3 is expanded;
Fig. 18 is a diagram showing a scissors clamp included in the EMR device in the embodiment of the present invention;
Fig. 19 is a diagram showing an insertion member included in the treatment instrument for EMR in the embodiment of the present invention;
Fig. 20 is a sectional view for explaining a forming operation by the insertion member shown in Fig. 19 of an injection part for inserting the separating and swelling member in the endoscopic mucosal resection carried out with the use of the EMR device having the treatment instrument for EMR of the embodiment of the present invention;
Fig. 21 is a sectional view for explaining the forming operation by the insertion member shown in Fig. 19 of the injection part for inserting the separating and swelling member in the endoscopic mucosal resection carried out with the use of the EMR device having the treatment instrument for EMR in the embodiment of the present invention;
Fig. 22 is a sectional view for explaining the forming operation by the insertion member shown in Fig. 19 of the injection part for inserting the separating and swelling member in the endoscopic mucosal resection carried out with the use of the EMR device having the treatment instrument for EMR in the embodiment of the present invention;
Fig. 23 is a sectional view for explaining the forming operation by the insertion member shown in Fig. 19 of the injection part for inserting the separating and swelling member in the endoscopic mucosal resection carried out with the use of the EMR device having the treatment instrument for EMR in the embodiment of the present invention;
Fig. 24 is a diagram showing a further modified example of the treatment instrument for EMR in the embodiment of the present invention;
Fig. 25 is a plan view of Fig. 20;
Fig. 26 is a plan view of Fig. 21;
Fig. 27 is a plan view of Fig. 22;
Fig. 28 is a plan view of Fig. 23; and
Fig. 29 is a diagram for explaining a conventional endoscopic mucosal resection.

### DETAILED DESCRIPTION OF THE PREFERRED EMBODIMENT

A treatment instrument for EMR and an EMR device as a preferred embodiment of the present invention will be described below with reference to the attached drawings throughout which like parts are designated by like reference numerals. In the specification, an endoscope represents a general instrument for observing, diagnosing and treating lesions in a body cavity directly by naked eyes or images.

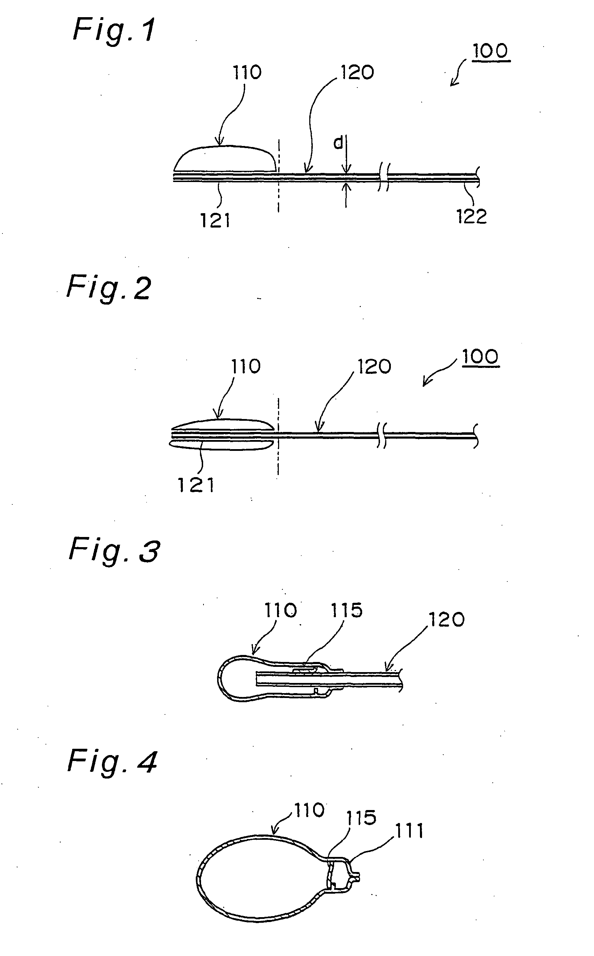

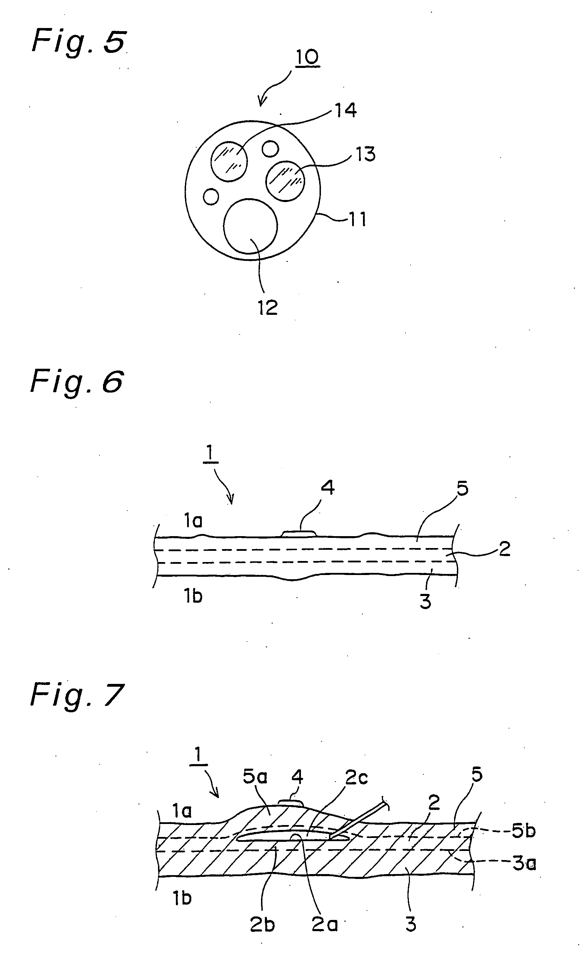

Fig. 1 shows a treatment instrument for EMR (Endoscopic Mucosal Resection) 100 of the present embodiment. The treatment instrument for EMR 100 comprises a separating and swelling member 110 and a first introduction member 120 in this embodiment. The separating and swelling member 110 is named also a balloon for EMR, which is inserted into a body cavity such as the stomach, large intestine, small intestine or esophagus through a clamp channel 12 of Fig. 5 included in an endoscope 10 such as shown in Fig. 16, and then inserted in a submucosal layer 2 below a lesion portion 4 present at a mucosal layer 5 of the body cavity, thereby separating and dissociating a portion to be dissociated 5a including the mucosal layer 5 where the lesion portion 4 is present, and a non-affected portion 2b within the submucosal layer 2 under the portion to be dissociated 5a which does not include the lesion portion, and also swelling the portion to be dissociated 5a including the lesion portion 4. The separating and swelling member 110 is connected to a leading end 121 of the first introduction member 120.

In concept, the above portion to be dissociated 5a means both of the mucosal layer 5 alone, and the mucosal layer 5 and also an upper layer part of the submucosal layer 2, the upper layer part being in the vicinity of a boundary to the mucosal layer 5. A reference numeral 13 in Fig. 5 indicates a lens for imaging the interior of the body cavity, and a reference numeral 14 denotes an illuminating part for the above imaging.

The separating and swelling member 110 can be attached to the first introduction member 120 to hold the leading end 121 of the first introduction member 120 as is indicated in Fig. 2.

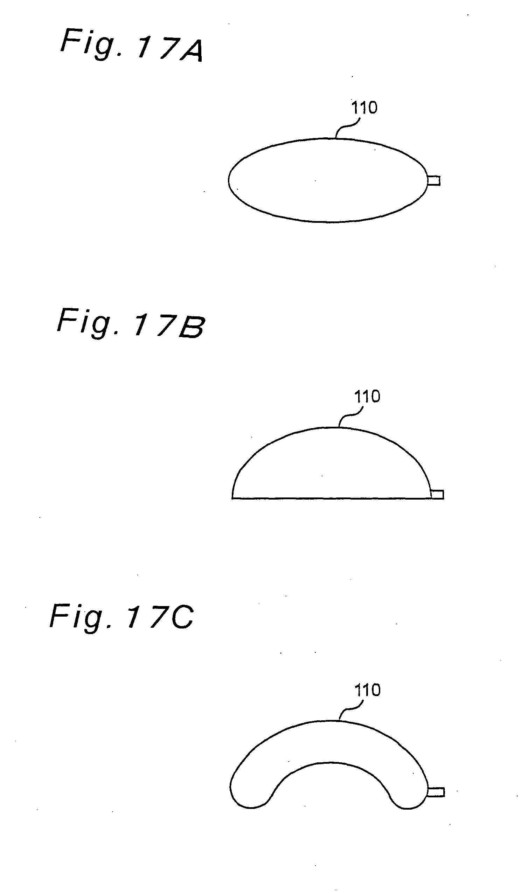

The separating and swelling member 110 as above is formed, for instance, in the shape of a bag and is formed of, e.g., a freely expansible and contractible elastic material such as rubber material to be expanded by a gas or a liquid supplied through the first introduction member 120, thereby performing the separation and dissociation, and furthermore making at least the lesion portion 4 swell. As is described later, the lesion portion 4 swollen by the separating and swelling member 110 is excised by a diathermy knife or the like together with the mucosal layer 5 in a periphery of the lesion portion 4, or the mucosal layer 5 and part of the submucosal layer 2. Therefore, the separating and swelling member 110 is preferably formed of a material with a heat resistance and a durability capable of resisting heat, sparks, and damage by a needle or the like at the time of the excising operation, for example, a rubber material such as silicone rubber, natural rubber or synthetic rubber, a styrene-based elastomer material, a urethane-based elastomer material, a thermoplastic material such as polyethylene, polypropylene or the like, vinyl chloride, or the like. A thickness of the separating and swelling member 110 is different from that of an insertion member 130 to be described later, is made uniform all over the total length, and can be formed to be not smaller than a maximum thickness of the insertion member 130. Moreover, since the separating and swelling member 110 is required to swell at least the lesion portion 4, and even the mucosal layer 5 including also the periphery of the lesion portion 4 and part of the submucosal layer 2, the separating and swelling member 110 should be conformed in size to the lesion portion 4, that is, to a size whereby at least the lesion portion 4 is swollen. As an example, the separating and swelling member 110 is a nearly circle of a diameter of approximately 50mm when expanded and has a total height (thickness) of approximately 5-10mm when expanded. The shape of the separating and swelling member 110 when expanded is not limited to the above nearly circular shape, and is adapted to match a shape of the lesion portion 4. For example, an elliptic shape shown in Fig. 17A, a semi circular shape shown in Fig. 17B, an arc shape shown in Fig. 17C or the like is conceivable.

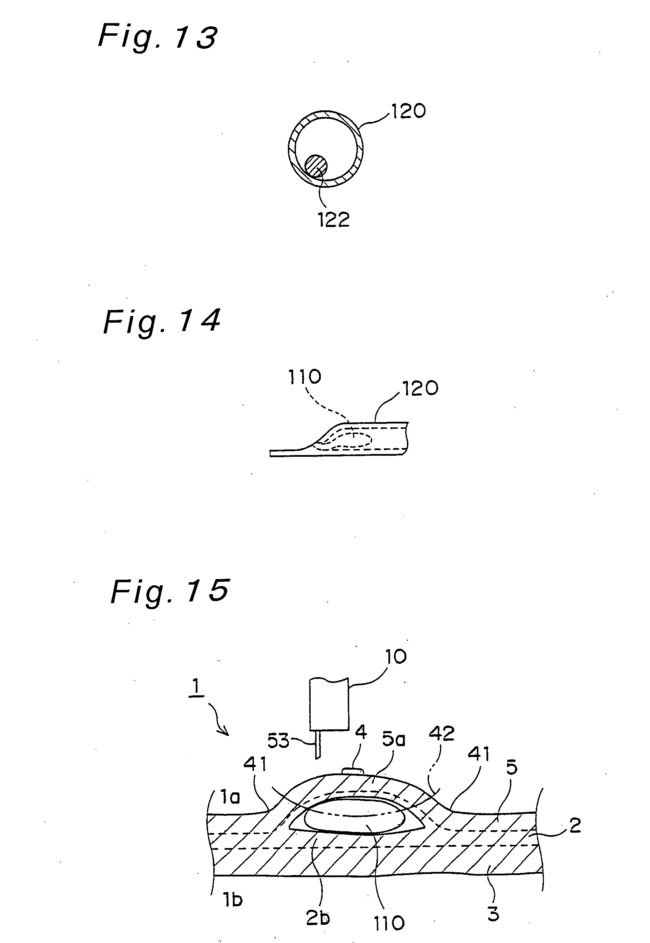

The first introduction member 120 is a tubular member having a diameter "d" of approximately 1.8mm and a length of approximately 2m to be able to pass through the clamp channel 12 of the endoscope 10. As mentioned earlier, the first introduction member 120 has its leading end 121 connected to the separating and swelling member 110 so that the gas or liquid can be injected to an inside of the bag-shaped separating and swelling member 110 through the first introduction member 120. The separating and swelling member 110 may be installed to the outside of the leading end of the first introduction member 120 in a state while shrunk,' or to the inside of the first introduction member 120 at the leading end. The gas to be injected is, for instance, air, and the liquid to be injected is, e.g., water, physiological saline, a gel of pharmaceutical solution or the like. Additionally, because of the need of sending the separating and swelling member 110 through the clamp channel 12 of the endoscope 10 and further inserting the separating and swelling member 110 into the submucosal layer 2 below the lesion portion 4, the first introduction member 120 can be formed of a material with a suitable hardness for supporting itself. Or, a guide member 122 having a suitable hardness such as a piano wire can be buried in the first introduction member 120 or laid along the first introduction member 120. The guide member 122 may be arranged within the first introduction member 120 as illustrated in Fig. 13.

Furthermore, the first introduction member 120 and the separating and swelling member 110 can adopt a detachable structure to each other. More specifically, it can be designed so that the separating and swelling member 110 is left in the submucosal layer 2 with expanded condition after it is expanded within the submucosal layer 2, and the first introduction member 120 is removed from the separating and swelling member 110 to be taken out solely from inside the body cavity to the outside of the patient body. In this case, for maintaining the expansion state of the separating and swelling member 110, for instance, an installation and removal mechanism 115 is fixed to the separating and swelling member 110 as shown in Fig. 3. As an example of the installation and removal mechanism 115, a check valve structure is employable for closing an introduction member insertion opening 111 of the separating and swelling member 110 simultaneously with the detachment of the first introduction member 120 from the separating and swelling member 110. The installation and removal mechanism 115 is not restricted to the check valve structure. For example, the installation and removal mechanism 115 can be formed in such structure that enables separation of the separating and swelling member 110 and the first introduction member 120 from each other while the expansion state of the separating and swelling member 110 is maintained by twisting the first introduction member 120 to the separating and swelling member 110. Alternatively, the installation and removal mechanism 115 can be arranged to the first introduction member 120 instead of the separating and swelling member 110, or to both of the separating and swelling member 110 and the first introduction member 120.

Although it is necessary to insert the leading end of the first introduction member 120 and the separating and swelling member 110 into the submucosal layer 2 as described above, if the leading end of the first introduction member 120 is sharpened, the straightforwardness of the leading end of the first introduction member 120 is intensified so much as to possibly send the separating and swelling member 110 to the muscular layer 3 below the submucosal layer 2 or to the body cavity outside 1b. Therefore, the leading end of the first introduction member 120 is preferably formed in a shape to weaken the straight-ahead state so that the first introduction member 120 and the separating and swelling member 110 are guided into the submucosal layer 2 without reaching the muscular layer 3 even when the leading end is inserted slantwise towards a direction of the muscular layer 3. As an example of the shape for weakening the straight-ahead state, a spatula-like shape as shown in Fig. 14 can be considered. Fig. 14 indicates a state in which the separating and swelling member 110 is arranged within the first introduction member 120.

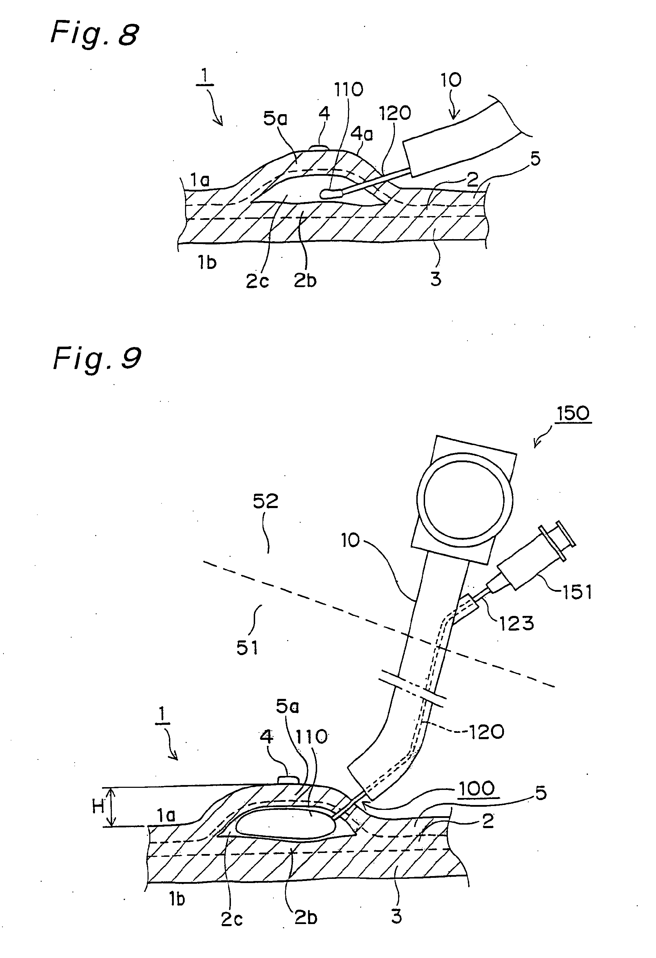

Referring now to Fig. 9, an EMR device 150 of the embodiment is equipped with the above-described treatment instrument for EMR 100, and the endoscope 10 through which the treatment instrument 100 is passed and which guides the treatment instrument 100 into the body cavity 1 inside a patient body 51. Moreover, an expansion tool 151 is connected to a rear end 123 of the first introduction member 120 of the treatment instrument for EMR 100 inserted in the endoscope 10. The rear end 123 is positioned outside a patient body 52 and extended to the outside of the endoscope 10. The expansion tool 151 expands the separating and swelling member 110 by supplying the gas or the liquid to the separating and swelling member 110 attached to the leading end 121 of the first introduction member 120. For the expansion tool 151, a syringe such as illustrated is convenient, but is not limited to the syringe.

A use method of the treatment instrument for EMR 100 and the EMR device 150 constituted as above, namely, the endoscopic mucosal resection carried out with the use of the treatment instrument for EMR 100 and the EMR device 150 will be described below.

In the first place, as is clear in Fig. 7, a puncture needle or the like is pierced through the endoscope 10 at a separating position 2a in the submucosal layer 2 located below the lesion portion 4 present at the mucosal layer 5 of the body cavity 1 shown in Fig. 6. For example, physiological saline or the like is injected from the puncture needle or the like. This is to facilitate insertion of the separating and swelling member 110 into the submucosal layer 2 below the lesion portion 4, which will be described later. The separating position 2a is any position in a thickness direction of the submucosal layer 2. The uppermost position of the separating position 2a is a boundary part 5b between the mucosal layer 5 and the submucosal layer 2, and the lowermost position is a boundary part 3a between the submucosal layer 2 and the muscular layer 3.

Subsequently a small hole is opened to reach the submucosal layer 2 with the diathermy knife or the like in a peripheral part 4a of the lesion portion through the endoscope 10. Then, the treatment instrument for EMR 100 is inserted in the clamp channel 12 of the endoscope 10, and furthermore the separating and swelling member 100 is inserted through the small hole into an injection part 2c where the physiological saline or the like is injected beforehand, as indicated in Figs. 8 and 9.

Thereafter, the gas or the liquid is supplied through the first introduction member 120 to the separating and swelling member 110 with the use of the expansion tool 151 connected to the rear end 123 positioned outside the patient body 52 among the first introduction member 120 of the treatment instrument for EMR 100 as is shown in Fig. 9. The separating and swelling member 110 is consequently expanded, thereby separating and dissociating between the portion to be dissociated 5a and the non-affected portion 2b from each other within the submucosal layer 2. Moreover, the expansion of the separating and swelling member 110 swells at least the lesion portion 4. The portion to be dissociated 5a includes the mucosal layer 5 where the lesion portion 4 is present, or the mucosal layer 5 and even part of the submucosal layer 2. The non-affected portion 2b is under the portion to be dissociated 5a and does not include the lesion portion 4. A height whereby the lesion portion 4 is swollen, that is, a height "H" from a surface of a non-swollen part of the mucosal layer 5 to a surface of a swollen part of the mucosal layer 5 is, e.g., approximately 5mm.

Since the portion to be dissociated 5a and the non-affected portion 2b are separated and dissociated from each other by expanding the separating and swelling member 110 within the submucosal layer 2 as described hereinabove, the connective tissue 7a at a dissociating part in the submucosal layer 2 described with reference to Fig. 29 can be cut by the expansion of the separating and swelling member 110, whereby the need of cutting the connective tissue 7a at the treatment time as in the conventional art is eliminated.

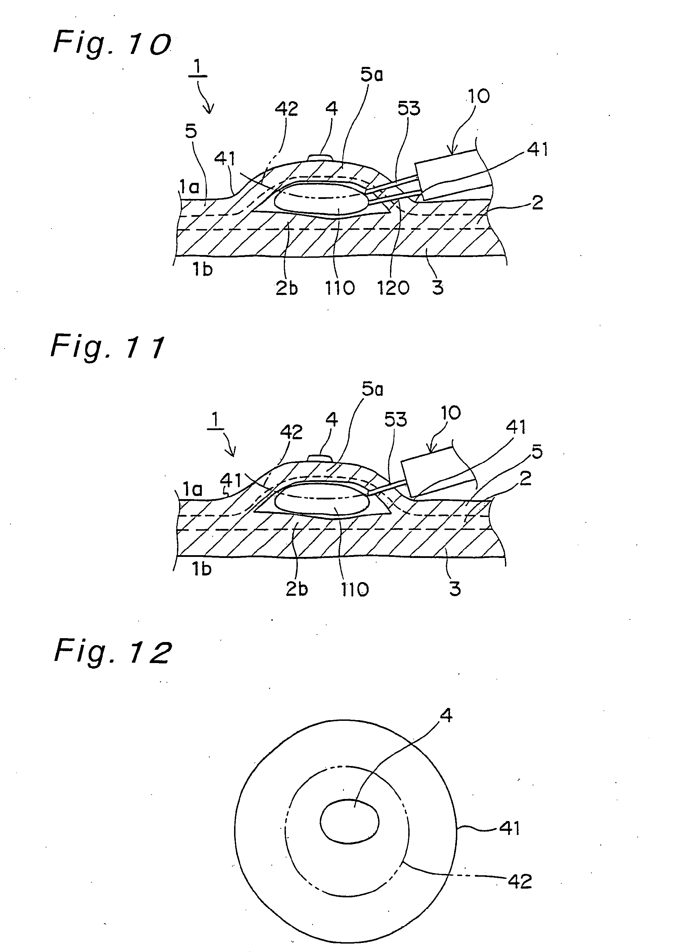

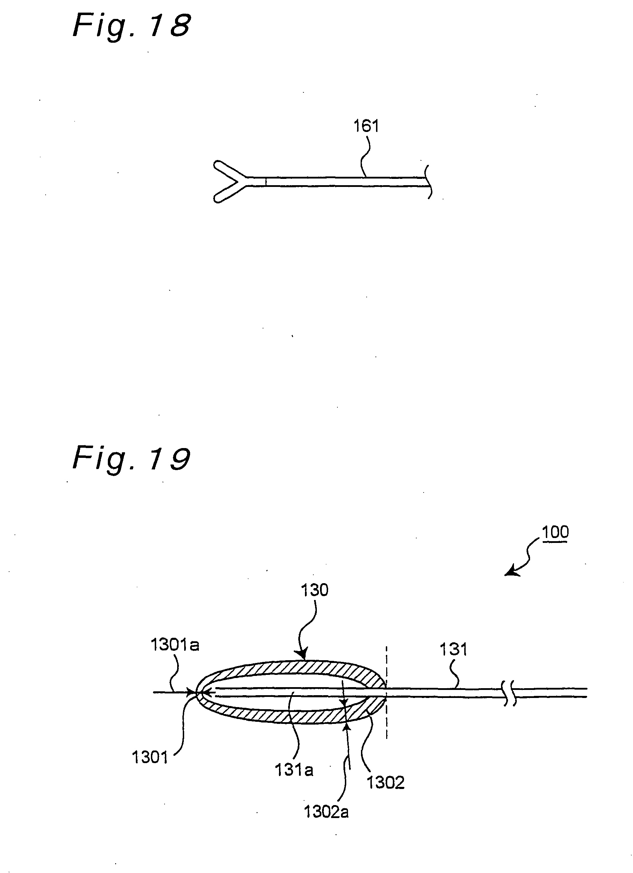

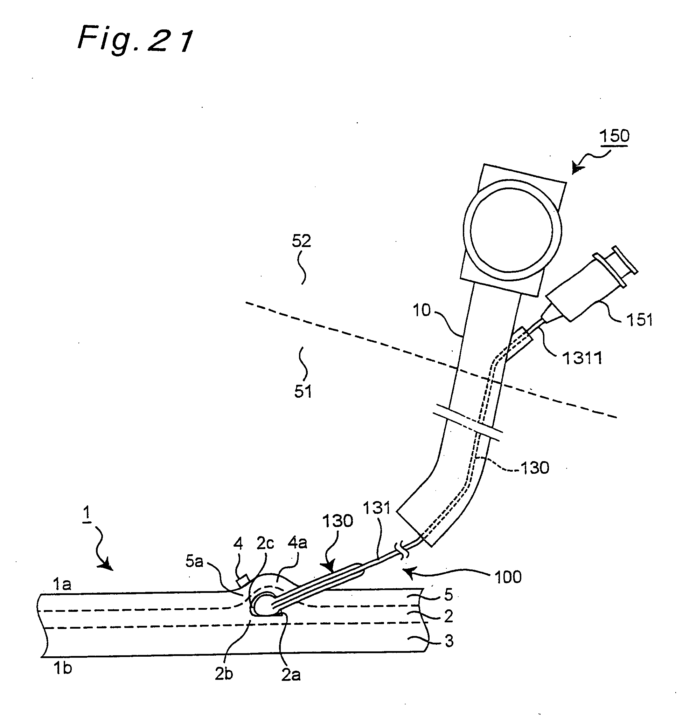

Subsequently, as is conceptually indicated by Fig. 12, the portion to be dissociated 5a including the lesion portion 4 is excised along an excision section 42 of the peripheral part of the lesion portion 4 among a swelling part 41 by the diathermy knife 53 or the like passed through the endoscope 10. At this time, although heat associated with the excision acts to the separating and swelling member 110, the heat resistance of the separating and swelling member 110 prevents damage to the same. Hence a swelling state of the lesion portion 4 is prevented from being lost during the treatment. At the time of the excision, a scissors clamp 161 shown in Fig. 18 can be used instead of the diathermy knife 53 or the like, or can be used together with the diathermy knife 53 or the like.

In the case where the installation and removal mechanism 115 is formed to at least one of the separating and swelling member 110 and the first introduction member 120, it becomes possible to separate the first introduction member 120 from the separating and swelling member 110 and pull out the first introduction member 120 to the outside of the patient body 52 as shown Fig. 11. Thereafter, the portion to be dissociated 5a including the lesion portion 4 is excised by at least either of the diathermy knife 53 or the like, and the scissors clamp 161 along the excision section 42.

In any case, the portion to be dissociated 5a which is excised is extracted together with the endoscope 10 including the separating and swelling member 110 and the first introduction member 120 to the outside of the patient body 52. In the case when the first introduction member 120 is taken out of the patient body 52 earlier, the separating and swelling member 110 is contracted after the affected portion is excised, and then taken to the outside of the patient body 52 together with the endoscope 10.

The endoscopic mucosal resection is now finished.

On the other hand, in the case where the above installation and removal mechanism 115 is formed neither to the separating and swelling member 110 nor to the first introduction member 120, the rear end 123 of the first introduction member 120 present outside the patient body is shut, thereby maintaining the expanded state of the separating and swelling member 110 in the submucosal layer 2. Thereafter, the endoscope 10 is once taken out of the patient body in a state with the separating and swelling member 110 and the first introduction member 120 being kept inserted in the patient body, and the first introduction member 120 is removed from within the endoscope 10. Then, the endoscope 10 is inserted again into the patient body, and at least one of the diathermy knife 53 or the like, and the scissors clamp 161 is passed through the emptied clamp channel 12 to excise the portion to be dissociated 5a in the manner as described above.

After the excision of the portion to be dissociated 5a, the excised portion 5a and the endoscope 10 are extracted from within the body cavity 1 to the outside of the patient body 52. The endoscopic mucosal resection is now finished.

In Figs. 10 and 11, the portion to be dissociated 5a is illustrated to be excised along the excision section 42 by the diathermy knife 53 or the like from a side face of the swelling part. However, since the connective tissue 7a in the injection part 2c is already cut through the expansion of the separating and swelling member 110 as described before, it is not necessary to dispose at least one of the diathermy knife 53 or the like, and the scissors clamp 161 at the side face of the swelling part which would deteriorate visibility of the lesion portion 4. Therefore, a leading end of the endoscope 10 can be disposed above the lesion portion 4 as indicated in Fig. 15 according to the treatment instrument for EMR 100 and the EMR device 150 of the present embodiment for improving the visibility of the lesion portion 4 and making the treatment easy and safe. Thus the portion to be dissociated 5a can be cut by at least one of the diathermy knife 53 or the like, and the scissors clamp 161 along the excision section 42 from above the lesion portion 4. Moreover, since the connective tissue 7a is already cut as mentioned above, the portion to be dissociated 5a can be disconnected from the non-affected portion 2b by simply cutting the portion to be dissociated 5a along the excision section 42. The portion to be dissociated 5a can be excised more easily and more safely in comparison with the conventional art.

According to the treatment instrument for EMR 100 and the EMR device 150 of the present embodiment as above, the portion to be dissociated 5a including the lesion portion 4, and the non-affected portion 2b not including the lesion portion 4 are separated and dissociated from each other, and further at least the lesion portion 4, preferably its peripheral part including the lesion portion 4 is swollen by using and expanding the separating and swelling member 110 of the treatment instrument for EMR 100. Therefore, the swelling is prevented from being lost or disappearing along with the start of cutting of the mucous membrane which is inherent in the conventional art, and the affected portion becomes unnecessary to be swollen for a plurality of the number of times. The possibility of generation of the complication as perforation and bleeding, and leaving the lesion portion 4 without removed can be almost eliminated.

Besides, since the connective tissue 7a in' the submucosal layer 2 is cut by the expansion of the separating and swelling member 110 within the submucosal layer 2 according to the present embodiment, in other words, since the separating and swelling member 110 is interposed between the portion to be dissociated 5a and the non-affected portion 2b, it becomes unnecessary to excise the portion to be dissociated 5a while the connective tissue 7a is being cut by the diathermy knife 53 or the like brought in from the side face of the swelling part. In consequence, the leading end of the endoscope 10 can be arranged to be opposite to the lesion portion 4 to improve the visibility of the lesion portion 4 by the endoscope 10, and the portion to be dissociated 5a can be excised by at least one of the diathermy knife 53 or the like, and the scissors clamp 161 from above the lesion potion 4. The treatment is thus facilitated as compared with the conventional art, with the possibility of generation of perforation, bleeding or the like complication being nearly avoided. The safety of the treatment can be improved and the burden to the patient can be reduced.

The treatment instrument for EMR 100 and the EMR device 150 of the embodiment enable easier and safer endoscopic mucosal resection in comparison with the conventional art, and accordingly an operation time can be greatly shortened as compared with the conventional art and the burden to the patient can be reduced.

While the separating and swelling member 110 of the treatment instrument for EMR 100 is expanded by the supply of the gas or liquid with the use of the expansion tool 151 in the above embodiment, for example, the separating and swelling member can be formed of a material with water absorption properties and be configured to expand by itself by absorbing water or the like in the submucosal layer 2.

In the above-described embodiment, by means of expanding the separating and swelling member 110, the separating and swelling member 110 is made to concurrently carry out the operation of separating and dissociating the portion to be dissociated 5a including the lesion portion 4 from the non-affected portion 2b without including the lesion portion 4, and the operation of swelling at least the lesion portion 4, preferably its peripheral part including the lesion portion 4. The separating and swelling member 110 is formed relatively large for performing the above swelling operation. Thus it is difficult in some cases to insert and introduce this large separating and swelling member 110 into the submucosal layer 2 located below the lesion portion 4. Therefore, the treatment instrument for EMR 100 is preferably further equipped with the insertion member 130 such as shown in Fig. 19, which is relatively small in size as compared with the separating and swelling member 110. The insertion member 130 is used before the separating and swelling member 110 is inserted into the submucosal layer 2. The insertion member 130 preliminarily forms the injection part 2c for the separating and swelling member 110 in the submucosal layer 2, and cuts the connective tissue 7a in the submucosal layer 2 along with the formation of the injection part 2c.

The insertion member 130 can be in any form so long as the above-described forming action for the injection part 2c can be achieved. A balloon-shaped form called IB balloon as described hereinbelow is adoptable as an one example of the insertion member 130. The insertion member 130 facilitates the insertion of the separating and swelling member 110 into the submucosal layer 2 and is arranged at a leading end 131a of a second introduction member 131 which supports the insertion of the insertion member 130 into the submucosal layer 2. Similar to the separating and swelling member 110, the insertion member 130 is shaped, e.g., like a bag and is formed of a freely expansible and contractible elastic material such as rubber material or elastomer material. After inserted into the submucosal layer 2, the insertion member 130 expands by a gas or a liquid supplied through the second introduction member 131. The insertion member 130 cuts the connective tissue 7a by the expansion as described above, separates and dissociates the portion to be dissociated 5a and the non-affected portion 2b from each other, thereby forming the injection part 2c where the separating and swelling member 110 is to be inserted.

Since the primary function of the insertion member 130 is to form the injection part 2c which facilitates the insertion of the separating and swelling member 110 to the submucosal layer 2 and the expansion of the separating and swelling member 110 in the submucosal layer 2 to prevent the separating and swelling member 110 from slipping off from the submucosal layer 2 at the time when the separating and swelling member 110 is expanded. Thus a range where the portion to be dissociated 5a and the non-affected portion 2b are separated and dissociated from each other by the insertion member 130 is small as compared with that by the separating and swelling member 110.

As described above, the insertion member 130 serves for part of the submucosal layer 2 to cut the connective tissue 7a, and separate and dissociate the portion to be dissociated 5a and the non-affected portion 2b from each other thereby forming the injection part 2c. From a viewpoint of smoothly proceeding the cutting and dissociating operation, at the expansion time by the supply of the gas or the like, the insertion member 130 is preferred to expand slimly and sequentially from a leading end 1301 to a rear end 1302 without expanding wide, rather than expand nearly uniformly all over the total length like the separating and swelling member 110. For realizing such expansion as above, according to the insertion member 130, a thickness 1301a at the leading end 1301 of the insertion member 130 is made smaller than a thickness 1302a at the rear end 1302. The insertion member 130 is formed to increase its thickness gradually from the leading end 1301 to the read end 1302. While the thickness at each part is determined in conformity with, for instance, a size of the lesion portion 4 or the like, by way of example, the thickness 1301a of the leading end 1301 can be set to approximately 150µm and the thickness 1302a of the rear end 1302 can be set to approximately 200µm.

In a case of performing the operation with the use of also the insertion member 130, the thickness of the separating and swelling member 110 can be made almost uniform over the total length thereof.

The insertion member 130 is not necessarily prepared in size to conform to the lesion portion 4. But the insertion member 130 has a size conformed to at least the lesion portion 4. The insertion member 130 is not required to be the same size as that of the separating and swelling member 110, and is smaller than the separating and swelling member 110 in many cases. By way of example, the insertion member 130 is approximately 1.5-2cm.

The second introduction member 131 is tubular in the same size with the same function as those of the above-described first introduction member 120. The guide member 122 described earlier can be attached to the second introduction member 131 for supporting the second introduction member 131. Moreover, substance to be injected into the second introduction member 131 is similar to that of the first introduction member 120. Specifically, the substance is, for example, air as the gas, and water, physiological saline, a gel of pharmaceutical solution or the like as the liquid.

The insertion member 130 is preferred to expand sequentially from its leading end 1301 towards the rear end 1302 as described above. For achieving the expansion state, it is preferable that the insertion member 130 is formed to the second introduction member 131 in a state in which a leading end of the second introduction member 131 is inserted to an inside of the slim insertion member 130 so that the leading end of the second introduction member 131 is disposed near the leading end 1301 of the insertion member 130, and only the rear end 1302 of the insertion member 130 is joined to the second introduction member 131, as shown in Fig. 19.

In the case of using both the separating and swelling member 110 and the insertion member 130, as is described later, since the insertion member 130 ends its service after dissociating the portion to be dissociated 5a and the non-affected portion 2b from each other by the expansion of the insertion member 130, subsequently the insertion member 130 is contracted and taken to the outside of the patient body together with the second introduction member 131. The insertion member 130 necessitates no installation and removal mechanism 115 accordingly.

Since the second introduction member 131 is inserted into the submucosal layer 2 similar to the first introduction member 120, the leading end of the second introduction member 131 is preferably formed into a shape whereby the straightforwardness is weakened, similar to the leading end of the first introduction member 120.

An endoscopic mucosal resection carried out with the use of the EMR device 150 having the treatment instrument for EMR 100 which further includes the insertion member 130 fitted to the leading end of the second introduction member 131 will be described below.

First, in reference to the lesion portion 4 present at the mucosal layer 5 of the body cavity 1 indicated in Fig. 6, the puncture needle or the like is pierced through the endoscope 10 to the separating position 2a in the submucosal layer 2 located below the lesion portion 4 as shown in Fig. 7. A small amount of, e.g., physiological saline or the like is injected to slightly swell the lesion portion 4. This swelling operation is to form a space for the insertion of the insertion member 130, which is described hereinbelow. The separating position 2a is any position in the thickness direction of the submucosal layer 2 as described before.

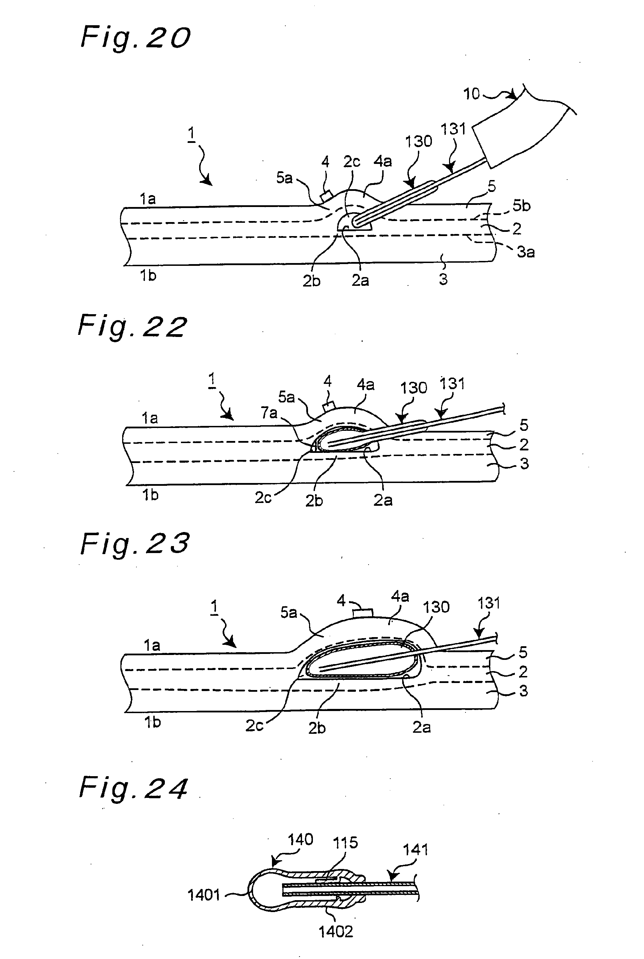

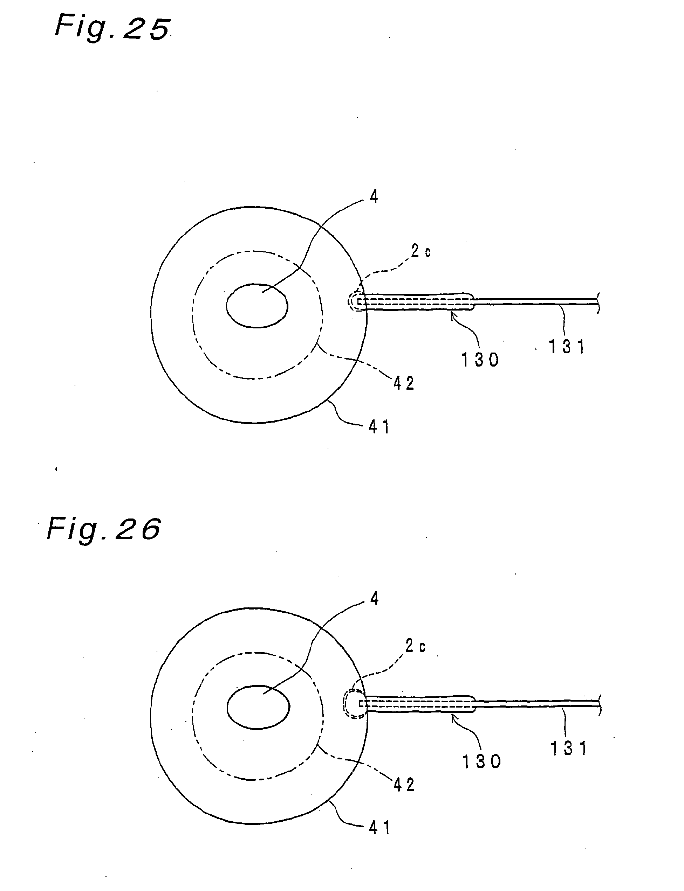

Next, a small hole reaching the submucosal layer 2 is opened at the peripheral part 4a of the lesion portion 4 by the diathermy knife or the like through the endoscope 10, and then the second introduction member 131 with the insertion member 130 fitted thereto among the treatment instrument for EMR 100 is inserted into the clamp channel 12 of the endoscope 10 as shown in Figs. 20 and 25. Furthermore, the leading end of the second introduction member 131, or the leading end 1301 of the insertion member 130 attached to the leading end of the second introduction member 131 is inserted through the small hole into the injection part 2c.

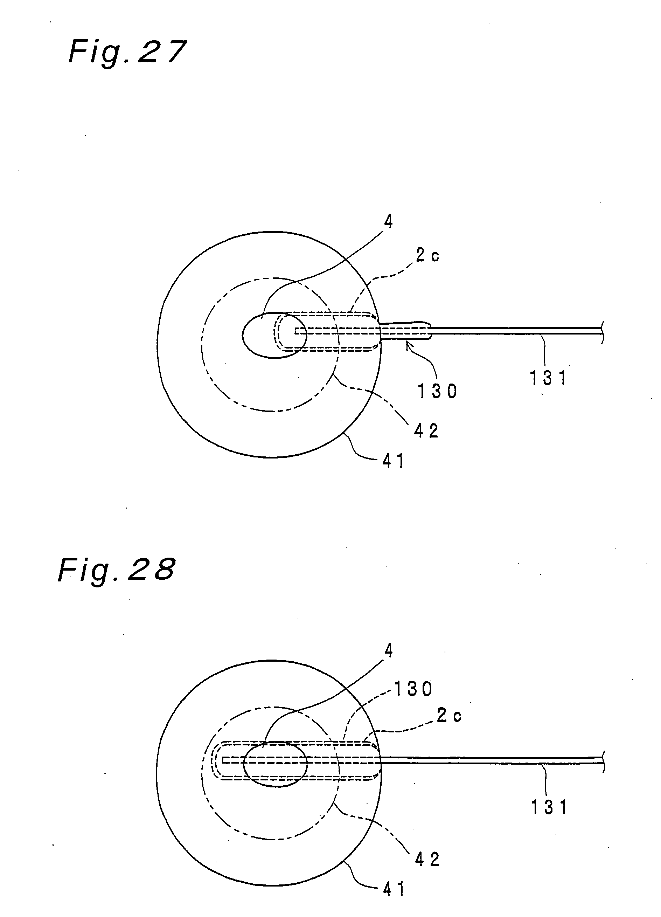

Subsequently, as shown in Figs. 21 and 26, the gas or the liquid is supplied into the insertion member 130 through the second introduction member 131 with the use of the expansion tool 151 connected to a rear end 1311 positioned outside the patient body 52 among the second introduction member 131. Since the insertion member 130 is formed to have the small thickness 1301a at the leading end 1301 thereof and have its thickness increased gradually from the leading end 1301 to the rear end 1302 thereof, the leading end 1301 is first expanded when the insertion member 130 is expanded. At this time, since the leading end 1301 of the insertion member 130 is inserted in the injection part 2c through the small hole, the expanded leading end 1301 becomes unable to pass through the small hole. In other words, the small hole acts like a stopper, preventing the insertion member 130 from being ejected to the body cavity 1 from in the submucosal layer 2 in accordance with the expansion of the insertion member 130. The insertion member 130 is sequentially expanded from the leading end 1301 towards the rear end 1302 as above. Therefore the insertion member 130 advances in the submucosal layer 2 while cutting the connective tissue 7a within the submucosal layer 2 in accordance with its expansion in a sequence from Figs. 22 to 23 and Figs. 27 to 28 and as is made clear from Figs. 22, 27 and 23, 28, thus forming the injection part 2c while the portion to be dissociated 5a, and the non-affected portion 2b under the portion to be dissociated 5a which does not include the lesion portion 4 are separated and dissociated from each other in the submucosal layer 2.

As above, the insertion member 130 expands slimly in its axis direction. Consequently a region to be separated and dissociated becomes slim as indicated in Figs. 26-28 and is prevented from being a nearly circular region centering the lesion portion 4. As above, the insertion member 130 forms a pilot tunnel for the insertion of the separating and swelling member 110.

At a time point when the above dissociating operation relative to the portion to be dissociated 5a in a size corresponding to at least the lesion portion 4 by the insertion member 130 ends, the expansion tool 151 is detached to release the expansion of the insertion member 130 and contract the insertion member 130. After the contraction, the second introduction member 131 and the insertion member 130 are taken out of the body through the endoscope 10.

After the second introduction member 131 and the insertion member 130 are taken out, the first introduction member 120 and the separating and swelling member 110 of the treatment instrument for EMR 100 are inserted into the clamp channel 12 of the endoscope 10 as illustrated in Figs. 8 and 9. The leading end of the first introduction member 120 and the separating and swelling member 110 are further inserted through the small hole into the injection part 2c within the submucosal layer 2 formed by the insertion member 130. Then the separating and swelling member 110 is expanded to carry out the endoscopic mucosal resection as described above. The swelling action by the separating and swelling member 110 separates and dissociates the portion to be dissociated 5a and the non-affected portion 2b from each other.

Since the pilot tunnel for the insertion of the separating and swelling member 110, namely, the injection part 2c is formed in the submucosal layer 2 by the insertion member 130 as above, the separating and swelling member 110 can be easily inserted into the submucosal layer 2 and is also prevented from slipping off from the submucosal layer 2 when the separating and swelling member 110 is expanded. The separating and swelling member 110 can easily swell at least the lesion portion 4, preferably, its peripheral part including the lesion portion 4. Therefore, in comparison with the case that the dissociating action is carried out by only the separating and swelling member 110, the treatment is facilitated, so that the possibility of generation of complication such as perforation and bleeding, and leaving the lesion portion 4 without removed is reduced more, the safety of the treatment is improved more, and the burden to the patient is lessened more.

Although the foregoing description relates to the case of using both the insertion member 130 and the separating and swelling member 110, it is possible to fabricate such a member for insertion, separating and swelling 140 as shown in Fig. 24 which is a combination of features of the insertion member 130 and the separating and swelling member 110. Similar to the insertion member 130, a thickness of the insertion and separating-swelling member 140 is gradually increased from its leading end 1401 towards a rear end 1402, and the installation and removal mechanism 115 is installed. The installation and removal mechanism 115 may be omitted from the insertion and separating-swelling member 140, and can be constructed in such forms as shown in Figs. 1 and 2.

The insertion and separating-swelling member 140 as above is attached to a leading end of a third introduction member 141, similar to the insertion member 130 and the separating and swelling member 110. The third introduction member 141 has the same structure and the same function as those of the first introduction member 120 and the second introduction member 131, and is an integrated member of the first introduction member 120 and the second introduction member 131.

According to the insertion and separating-swelling member 140, both the separating and dissociating operation for the portion to be dissociated 5a and the non-affected portion 2b, and the swelling operation for the portion to be dissociated 5a can be carried out by one member, whereby the operation time can be shortened and the burden associated with the operation to the patient can be reduced.

The present invention is applicable to treatment instruments for EMR for use in endoscopic mucosal resection, and EMR devices with the treatment instrument for EMR.

Although the present invention has been fully described in connection with the preferred embodiment thereof with reference to the accompanying drawings, it is to be noted that various changes and modifications are apparent to those skilled in the art. Such changes and modifications are to be understood as included within the scope of the present invention as defined by the appended claims unless they depart therefrom.

## Claims

1. A treatment instrument for EMR, which comprises:
a separating and swelling member (110) configured to be inserted in a submucosal layer (2) located below a lesion portion (4) present at a mucosal layer (5) of a body cavity (1) after inserted in the body cavity through an endoscope (10) so as to separate and dissociate a portion to be dissociated (5a) where the lesion portion is present and a non-affected portion (2b) under the portion to be dissociated without including the lesion portion from each other, and also swell the lesion portion; and
a first introduction member (120) having the separating and swelling member connected to a leading end (121) of the first introduction member, which is configured to support the insertion of the separating and swelling member into the submucosal layer.

2. The treatment instrument for EMR according to Claim 1, wherein the separating and swelling member is formed in a shape of a bag and of a material which expands by a gas or a liquid supplied through the first introduction member, the separating and swelling member having a size for swelling the lesion portion by the expansion.

3. The treatment instrument for EMR according to Claim 1 or 2, wherein the separating and swelling member is formed of a material having a heat resistance capable of resisting heat at a time of excising the swollen lesion portion.

4. The treatment instrument for EMR according to any one of Claims 1-3, wherein the treatment instrument has an installation and removal mechanism (115) configured to separate the first introduction member from the separating and swelling member while the swelling state of the lesion portion by the separating and swelling member is maintained.

5. The treatment instrument for EMR according to any one of Claims 1-4, which further comprises:
an insertion member (130) as a member configured to facilitate the insertion of the separating and swelling member into the submucosal layer, which is configured to be inserted in the submucosal layer and dissociate the portion to be dissociated where the lesion portion is present and the non-affected portion under the portion to be dissociated without including the lesion portion from each other so as to form an injection part (2c) where the separating and swelling member is to be inserted; and
a second introduction member (131) with the insertion member attached to a leading end (131a) of the second introduction member, which is configured to support the insertion of the insertion member into the submucosal layer.

6. The treatment instrument for EMR according to Claim 5, wherein the insertion member is integrally formed with the separating and swelling member, and the first introduction member and the second introduction member are integrally formed in one body.

7. An EMR device, which comprises:
a treatment instrument for EMR (100) including:
a separating and swelling member (110) configured to be inserted in a submucosal layer (2) located below a lesion portion (4) present at a mucosal layer (5) of a body cavity (1) after inserted in the body cavity through an endoscope (10) so as to separate and dissociate a portion to be dissociated (5a) where the lesion portion is present and a non-affected portion (2b) under the portion to be dissociated without including the lesion portion from each other, and also swell the lesion portion; and
a first introduction member (120) having the separating and swelling member connected to a leading end (121) of the first introduction member, which is configured to support the insertion of the separating and swelling member into the submucosal layer; and
the endoscope (10) to be inserted in the body cavity (1) from the body outside, which is configured to guide the treatment instrument for EMR passed therethrough into the body cavity.

8. The EMR device according to Claim 7, wherein the separating and swelling member is formed in a shape of a bag and of a material which expands by a gas or a liquid supplied through the first introduction member, the separating and swelling member having a size for swelling the lesion portion by the expansion.

9. The EMR device according to Claim 8, wherein the treatment instrument for EMR has an installation and removal mechanism (115) configured to separate the first introduction member from the separating and swelling member while the swelling state of the lesion portion by the separating and swelling member is maintained.

10. The EMR device according to any one of Claims 7-9, which further comprises an expansion tool (151) connected at the body outside to the first introduction member (120) which is to support the separating and swelling member (110) as a guiding member that constitutes the treatment instrument for EMR and swells the lesion portion (4) present at the mucosal layer (5) of the body cavity, and to inject a gas or a liquid to the separating and swelling member so that the separating and swelling member is expanded for swelling the lesion portion.

11. The EMR device according to Claim 10, wherein the treatment instrument for EMR further includes an insertion member (130) as a member configured to facilitate the insertion of the separating and swelling member into the submucosal layer, which is configured to be inserted in the submucosal layer and dissociate the portion to be dissociated (5a) where the lesion portion is present and the non-affected portion (2b) under the portion to be dissociated without including the lesion portion from each other so as to form an injection part (2c) where the separating and swelling member is to be inserted; and
an expansion tool (151) connected at the body outside to the first introduction member (120) which is to support the separating and swelling member (110) as a guiding member that constitutes the treatment instrument for EMR and swells the lesion portion (4) present at the mucosal layer (5) of the body cavity, and to inject a gas or a liquid to the separating and swelling member so that the separating and swelling member is expanded for swelling the lesion portion, while the expansion tool is also connected at the body outside to a second introduction member (131) which is to support the insertion member as a guiding member that constitutes the treatment instrument for EMR, and to inject a gas or a liquid to the insertion member so that the insertion member is expanded.
